# EUROPÄISCHE PATENTSCHRIFT

(11) **EP 3 793 428 B1**
(45) Veröffentlichungstag und Bekanntmachung des Hinweises auf die Patenterteilung: **03.04.2024**
(21) Anmeldenummer: 19729617.1
(22) Anmeldetag: 17.05.2019
(51) Int. Cl.: A61B 5/00, G06F 3/00, G16H 50/00, A61B 50/00

(54) **MULTIFUNKTIONSGEHÄUSE FÜR EINE MESSVORRICHTUNG**
MULTI-FUNCTION HOUSING FOR A MEASURING DEVICE
BOÎTIER MULTIFONCTION POUR UN DISPOSITIF DE MESURE

(30) Priorität: 17.05.2018 DE 102018003984
(43) Veröffentlichungstag der Anmeldung: 24.03.2021
(73) Patentinhaber: Flore, Ingo, 44141 Dortmund (DE)
(72) Erfinder: CHO, Ok-Kyung, 58239 Schwerte (DE); KIM, Yoon Ok, 58239 Schwerte (DE)
(74) Vertreter: Schneiders & Behrendt Bochum
(86) Internationale Anmeldenummer: PCT/EP2019/062841
(87) Internationale Veröffentlichungsnummer: WO 2019/219931

(56) Entgegenhaltungen:
- CN-A- 102 202 703
- CN-U- 205 054 439
- US-A1- 2011 112 418
- US-A1- 2014 316 223

## Beschreibung

Die Erfindung betrifft ein Multifunktionsgehäuse mit einer entnehmbar darin angeordneten medizinischen Messeinheit mit integrierter Sensorik, welches einen Gehäusekörper und einen beweglichen Deckel aufweist, wobei der Gehäusekörper durch den Deckel verschließbar ist. Außerdem betrifft die Erfindung ein Verfahren zur Durchführung einer Messung von physiologischen Parametern und zur Analyse der Parameter unter Verwendung eines Multifunktionsgehäuses.

Solche Gehäuse für transportable elektrische Messeinheiten sind bekannt und werden eingesetzt, um die darin befindlichen Messeinheiten sicher aufzubewahren und beim Transport gegen Stöße und Umwelteinflüsse wie Staub zu schützen. Gehäuse zum Laden von elektrische Messeinheiten sind auch aus US 2014/316223 A1, US 2011/112418 A1 oder CN 102 202 703 A bekannt.

Kompakte und transportable medizinische Messeinheiten erlauben es Anwendern, physiologische Parameter ihres Körpers im Alltag mit geringem Aufwand zu überwachen und liefern daher wichtige Informationen über den Gesundheitszustand des Anwenders. Die entstehenden Daten können vom Benutzer selbst oder in Zusammenarbeit mit medizinischem Personal ausgewertet werden und tragen zur Gesunderhaltung des Anwenders bei.

Die Nützlichkeit und Alltagstauglichkeit einer solchen medizinischen Messeinheit hängt neben der eigentlichen Messfunktion von verschiedenen Faktoren ab, die im Entwurf der Messeinheit berücksichtigt werden müssen: Sollen beispielsweise Messungen auch unterwegs durchgeführt werden, so ist es wichtig, dass die Messeinheit einfach und sicher aufzubewahren und zu transportieren ist. Wird die medizinische Messeinheit während der Messung in der Hand gehalten, dann sind außerdem ein geringes Volumen und Gewicht der Messeinheit wünschenswert.

Insbesondere wenn die Messeinheit elektrisch leitfähige Gehäuseteile, z.B. Elektroden aufweist, mit denen der Anwender in Berührung kommen kann, stellt dies auch hohe Anforderungen an die elektrische Sicherheit der Messeinheit dar. Dies trifft besonders dann zu, wenn die Messeinheit zusätzlich über externe Schnittstellen geladen werden kann oder Daten übertragen werden sollen. Die daraus resultierenden notwendigen Maßnahmen zur Einhaltung der elektrischen Sicherheit führen zu einer Vergrößerung der Schaltung der Messeinheit und damit zu einer Volumenvergrößerung.

Es ist daher Aufgabe der Erfindung, ein Multifunktionsgehäuse der eingangs genannten Art dahingehend weiterzuentwickeln, dass die Messeinheit in dem Multifunktionsgehäuse nicht nur geschützt transportiert und aufbewahrt werden kann, sondern auch weitere Funktionalitäten der Messeinheit durch das Multifunktionsgehäuse übernommen werden oder mit Hilfe des Multifunktionsgehäuses durchgeführt werden können, sodass die Messeinheit auch bei vielfältiger Funktionalität kompakt und damit einfach handhabbar sowie elektrisch sicher bleibt.

Zur Lösung der Aufgabe schlägt die Erfindung ausgehend von einem Multifunktionsgehäuse der eingangs genannten Art vor, dass das Multifunktionsgehäuse über eine integrierte Schaltung verfügt und eine oder mehrere Schnittstellen aufweist, über die die Messeinheit Daten mit weiteren Geräten austauschen oder über die die Messeinheit aufgeladen werden kann. Die Erfindung schlägt dabei vor, dass das Laden des Akkus und der Datentransfer nur bei geschlossenem Multifunktionsgehäuse möglich sind.

Die Erfindung wird in den Patentansprüchen definiert.

Die integrierte Schaltung kann je nach Anforderung ausgelegt werden, also je nachdem welche Funktionen sie ausführen und/oder übernehmen soll. Im einfachsten Fall übernimmt die integrierte Schaltung lediglich die Funktion einer Adapterschnittstelle und leitet beispielsweise Daten und/oder elektrische Leistung durch. Die integrierte Schaltung kann aber auch als leistungsfähiger Mikrocontroller ausgebildet sein. Hierdurch ist es möglich, dass die Elektronik der Messeinheit eher leistungsschwach ausgelegt ist und lediglich dazu eingerichtet ist, die Messdaten aufzunehmen und temporär zu speichern. Alle weiteren Funktionen, wie beispielsweise die Datenaufbereitung und ähnliches, übernimmt dann der Mikrocontroller im Multifunktionsgehäuse.

Das Multifunktionsgehäuse weist eine Ladeschnittstelle für die kompakte Messeinheit auf. Hierdurch ist die Messeinheit auf einfache

Art und Weise aufladbar, wenn sie im Multifunktionsgehäuse angeordnet ist.

Es ist auch vorteilhaft, wenn das Multifunktionsgehäuse eine Datenkommunikationsschnittstelle für die Messeinheit aufweist. Über diese Schnittstelle kann die Messeinheit und/oder das Multifunktionsgehäuse mit externen Einheiten kommunizieren und beispielsweise die gemessenen Daten an einen übergeordneten Server oder ähnliches weiterleiten, auch wenn sich die Messeinheit im Multifunktionsgehäuse befindet.

Die Datenkommunikations- und Ladeschnittstelle kann auch als physische Einheit, beispielsweise als USB-Schnittstelle, ausgebildet sein. Die Messeinheit selbst muss somit nicht mehr über eigene Standard-Konnektoren verfügen, sondern lediglich über auf das Multifunktionsgehäuse abgestimmte Konnektoren, was der Kompaktheit der Messeinheit zu Gute kommt. Dies hat den weiteren Vorteil, dass keine Ladegeräte mit falscher Ladeleistung angeschlossen werden können, durch welche die oftmals empfindliche Elektronik und Sensorik der Messeinheit beschädigt oder zerstört werden könnte. Außerdem wird verhindert, dass die Messeinheit an andere, nicht dem Anwendungszweck entsprechende elektronische Geräte angeschlossen wird, die die elektrische Sicherheit des Anwenders gefährden könnten.

Eine Weiterbildung der Erfindung sieht vor, dass die Datenkommunikationsschnittstelle des Multifunktionsgehäuses als Bluetooth-Schnittstelle ausgebildet ist. Durch diese Maßnahme lässt sich bequem aus der näheren Umgebung mit der im Multifunktionsgehäuse befindlichen Messeinheit kommunizieren.

Eine bevorzugte Ausführungsform sieht vor, dass das Multifunktionsgehäuse ein transparentes Fenster aufweist. Hierdurch kann beispielsweise eine Anzeige an der Messeinheit (soweit vorhanden) auch im geschlossenen Zustand abgelesen werden. Die Zustandsdaten können beispielsweise Statusinformationen wie den Ladezustand der Messeinheit oder den Transferstatus im Falle eines Datentransfers über eine externe Schnittstelle umfassen.

Vorzugsweise weist das transparente Fenster eine Lupenfunktion auf, sodass auch ein Bediener mit eingeschränkter Sehstärke die Anzeige oder die Statusinformation der Messeinheit ohne Weiteres ablesen kann.

Der Deckel kann, z.B. zum Schutz des Gehäuseinneren vor Umgebungslicht, jedoch auch einen Filter für bestimmte Anteile des elektromagnetischen Spektrums enthalten oder vollständig intransparent sein. In diesem Fall sind eine eigenständige Anzeige sowie zusätzliche Bedienelemente, die in das Multifunktionsgehäuse integriert sind, nützlich, da die Anzeige der Messeinheit in diesem Fall nicht genutzt werden kann.

Weiterhin kann der Deckel beispielsweise mittels eines Klappmechanismus oder mittels eines Schiebmechanismus geöffnet und verschlossen werden. Diese Mechanismen sind besonders einfach zu bedienen. Am Deckel sind Mittel vorgesehen, um zu detektieren, ob das Multifunktionsgehäuse verschlossen ist. Hierdurch wird das Laden der Messeinheit und/oder die Kommunikation mit der Messeinheit nur ermöglicht, wenn das Multifunktionsgehäuse geschlossen ist. Dies stellt ebenfalls ein Mittel zur Erhöhung der elektrischen Sicherheit dar, da elektrisch leitende Gehäuseteile der Messeinheit im geschlossenen Zustand des Multifunktionsgehäuses nicht versehentlich berührt werden können.

Weiterhin kann es vorteilhaft sein, wenn das Multifunktionsgehäuse über eine Ton- und/oder Sprachausgabe verfügt. Somit können die Messergebnisse der Messeinheit über das Multifunktionsgehäuse auch auditiv wahrgenommen werden. Des Weiteren können beispielsweise Anwendungs- oder Warnhinweise über die Sprachausgabe ausgegeben werden.

Eine Weiterbildung sieht vor, dass am Multifunktionsgehäuse Mittel zur Klimatisierung des Innenraums, z.B. Lüfter, des Multifunktionsgehäuses vorgesehen sind. Durch diese Maßnahme kann die Messeinheit und insbesondere deren Sensorik vor zu großen Temperaturschwankungen geschützt werden, sodass die Einsatzfähigkeit der Messeinheit nicht durch die Temperatur beeinflusst wird.

Von ganz besonderem Vorteil ist es, wenn in dem Multifunktionsgehäuse Mittel zur Erzeugung eines optischen, thermischen oder elektrischen Referenzsignals vorgesehen sind. Die Referenzsignale dienen dazu, die Messeinheit zu kalibrieren und/oder Funktionstests durchzuführen. Bei den meisten medizinischen Messeinheiten sind die regelmäßige Kalibration und Funktionstests notwendig und teilweise sogar vorgeschrieben. Ohne diese Maßnahmen drohen nämlich Fehlmessungen, die erhebliche Folgen haben können. Daher ist es bei medizinischen Messeinheiten vorgeschrieben, soweit es zur Sicherstellung gültiger Ergebnisse erforderlich ist, dass diese in festgelegten Abständen bzw. vor dem Gebrauch anhand von Messnormalen, die auf internationale oder nationale Messnormale zurückgeführt werden, kalibriert oder verifiziert werden (vgl. ISO 13485).

Im Folgenden wird die Erfindung anhand von Zeichnungen und Ausführungsbeispielen näher erläutert. Es zeigen:
- Figur 1:: Schematisch eine Ansicht eines erfindungsgemäßen Multifunktionsgehäuses;
- Figur 2a/b:: Schematisch weitere Ansichten des Multifunktionsgehäuses;
- Figur 3:: Ein Blockschaltbild eines erfindungsgemäßen Multifunktionsgehäuses;
- Figur 4:: Schematisch eine Ansicht eines Multifunktionsgehäuses mit Metallkontakten zur Applikation von elektrischen Referenzsignalen;
- Figur 5:: Schematisch eine Ansicht eines Hilfsmittels zur Applikation von elektrischen Referenzsignalen bei Messeinheiten mit Sensoren im Inneren;
- Figur 6:: Ablaufdiagramm für den Einsatz eines erfindungsgemäßen Multifunktionsgehäuses bei Verwendung in der Produktion.

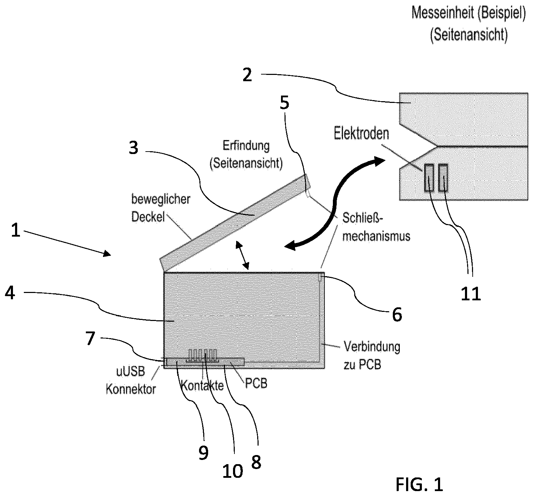

In Figur 1 ist ein Multifunktionsgehäuse 1 dargestellt, welches eine kompakte medizinische Messeinheit 2 in sich aufnimmt.

Das Multifunktionsgehäuse 1 verfügt über einen beweglichen, aufklappbaren Deckel 3 und einen Gehäusekörper 4. Wenn der Deckel 2 geöffnet ist, kann die medizinische Messeinheit in das Multifunktionsgehäuse 1 eingelegt werden. Der Innenraum des Multifunktionsgehäuses 1 ist auf die Größe der Messeinheit 2 angepasst und mit einem weichen Material ausgekleidet, das Stöße beim Transport dämpft. Die hier dargestellte Form ist nur exemplarisch. Es sind auch jedwede andere Formgestaltungen denkbar. Beispielsweise kann für eine zylinderförmige Messeinheit auch ein zylinderförmiges Multifunktionsgehäuse konstruiert werden. Die äußere Form der Erfindung muss jedoch nicht notwendigerweise durch die Form der Messeinheit bedingt sein, sondern eine Anpassung kann z.B. auch aus rein ästhetischen Gründen erfolgen.

Der bewegliche Deckel 3 verfügt über einen Kontaktstift 5, der ein entsprechendes Gegenstück 6 am Gehäusekörper 4 hat. Über den Kontaktstift 5 und das entsprechende Gegenstück 6 kann elektrisch festgestellt werden, ob der Deckel 3 geöffnet oder geschlossen ist. Dadurch kann sichergestellt werden, dass bestimmte Funktionen (bspw. Laden des Akkus, Datenübertragung) nur dann stattfinden, wenn der Deckel 3 geschlossen ist.

Das Multifunktionsgehäuse 1 verfügt über einen von außen zugänglichen Konnektor 7 für eine externe Schnittstelle (z.B. Micro-USB), über welche die Messeinheit sowohl geladen werden kann als auch eine Datenübertragung zwischen Messeinheit 2 und Umgebung möglich ist.

Durch die Verwendung einer gängigen Schnittstelle wie Micro-USB am Multifunktionsgehäuse 1, nicht aber an der Messeinheit 2, können das Aufladen des Akkus sowie der Datenaustausch mit der Umgebung einfach realisiert werden, ohne die Sicherheit des Anwenders oder der Messeinheit 2 zu gefährden.

Das Multifunktionsgehäuse 1 verfügt über eine integrierte Schaltung 8, die hier auf einem PCB (Printed Circuit Board) 9 angeordnet ist. Das PCB 9 enthält die notwendigen Schaltungsteile, um folgende Funktionalität zu realisieren:
- Galvanische Trennung zwischen externem Anschluss und Messeinheit 2;
- Aufladen des Akkus in der Messeinheit 2;
- Datentransfer zwischen Messeinheit 2 und Umgebung;
- Feststellung des Zustands des Deckels 3 (geöffnet/geschlossen);
- Unterbinden von Datentransfer und Aufladen des Akkus bei geöffnetem Deckel 3.

Zur Realisierung der Kommunikation zwischen Messeinheit 2 und der integrierten Schaltung 8 im Multifunktionsgehäuse 1 ohne gängigen Konnektor werden Metallkontakte 10 verwendet (z.B. Federkontakte), die ein entsprechendes Gegenstück auf der Messeinheit 2 haben. Die Messeinheit 2 weist weiterhin Messelektroden 11 auf für Bioimpedanz- oder ECG-Messungen.

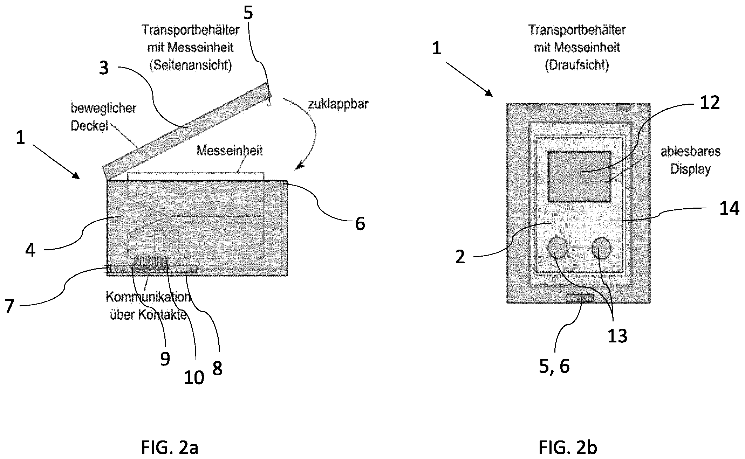

Figur 2a und 2b zeigen das Multifunktionsgehäuse 1 mit der entnehmbar darin angeordneten Messeinheit 2 in einer Seitenansicht bzw. in Draufsicht. Die Messeinheit 2 weist ein Display 12 sowie Bedienknöpfe 13 auf. Der Deckel 3 des Multifunktionsgehäuses 1 ist mit einem transparenten Fenster 14 versehen, sodass das Display 12 auch bei geschlossenem Deckel 3 ablesbar ist.

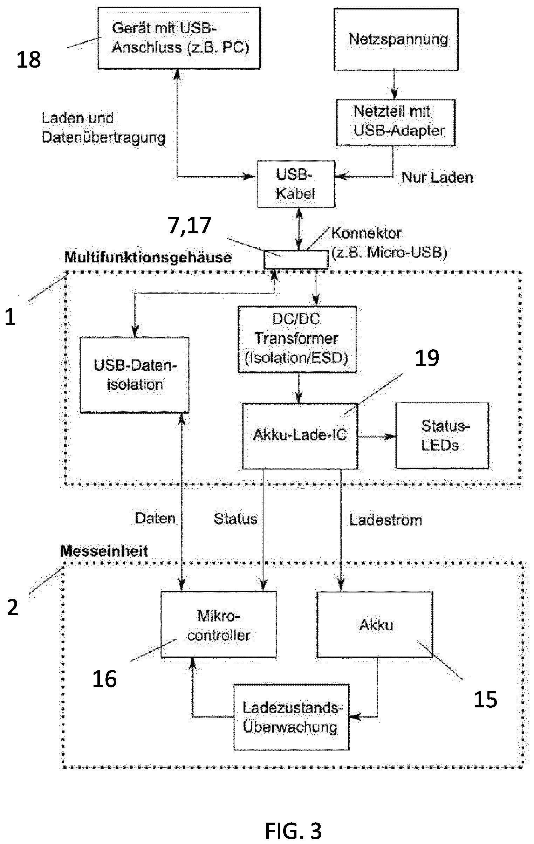

Figur 3 zeigt ein Blockschaltbild eines Multifunktionsgehäuses 1, in dem erfindungsgemäß eine Messeinheit 2 angeordnet ist. Die integrierte Schaltung 8 ist elektrisch mit dem Akku 15 der Messeinheit 2 verbunden und steuert deren Ladung. Hierdurch wird sichergestellt, dass nur der korrekte Ladestrom verwendet wird und die Messeinheit 2 keinen Schaden aufgrund einer falsch angeschlossenen oder falsch dimensionierten Ladevorrichtung nimmt. Ein Akku-Lade-IC 19 kann sich wie in Figur 3 gezeigt in der integrierten Schaltung 8 befinden, jedoch auch in der Messeinheit 2 selber; dies hat keinen Einfluss auf die Gesamtfunktionalität des Aufbaus. Der Akku-Lade-IC 19 steuert darüber hinaus Status-LEDs im Multifunktionsgehäuse 1, die den Ladezustand signalisieren. Sofern die Messeinheit 2 während des Ladevorgangs eingeschaltet ist, kann der Mikrocontroller 16 der Messeinheit 2 ebenfalls den Ladezustand des Akkus 15 überwachen und z.B. auf dem Display 12 der Messeinheit darstellen. Schließlich dient der Mikrocontroller 16 der Steuerung der Messeinheit 2 beim Messvorgang und nimmt die Messdaten auf. Erfindungsgemäß kann entweder der Mikrocontroller 16 die Daten weiterverarbeiten, oder diese Funktion wird von der integrierten Schaltung 8 des Multifunktionsgehäuses 1 durchgeführt.

Das Multifunktionsgehäuse 1 verfügt über eine externe Schnittstelle, nämlich einen USB-Anschluss 17. Über den USB-Anschluss 17 kann der Ladestrom für die Messeinheit eingespeist werden und auch mit weiteren Einheiten in der Umgebung kommuniziert werden. Hierbei kann es sich beispielsweise um einen Personal Computer 18 handeln. Zur Kommunikation ist auch eine hier nicht dargestellte, drahtlose Datenschnittstelle denkbar, beispielsweise eine Bluetooth-Schnittstelle. Wie zuvor bereits erwähnt, kann ein Großteil der Rechenleistungskapazität der Messeinheit 2 durch die integrierte Schaltung 8 im Multifunktionsgehäuse 1 übernommen werden. Hierdurch ist es möglich, dass die Messeinheit 2 sehr kompakt und damit gut handhabbar bleibt.

Durch die Kombination des Multifunktionsgehäuses 1 mit der Messeinheit 2 werden folgende Ziele erreicht:
Die Messeinheit 2 kann im eingelegten Zustand sowie bei geschlossenem Deckel 3 sicher transportiert und aufbewahrt werden.

Die elektrische Sicherheit der Messeinheit 2 wird erhöht, weil die Messeinheit 2 keinen gängigen Konnektor für das Aufladen des Akkus 15 und den Datentransfer haben muss. Die Kommunikation zwischen der Messeinheit 2 und dem Multifunktionsgehäuse 1 erfolgt nur über Metallkontakte. Der Anwender kann die Messeinheit 2 dementsprechend nicht versehentlich an eine Spannungsquelle anschließen und dadurch seine Sicherheit gefährden.

Die elektrische Sicherheit des Gesamtsystems wird erhöht, weil eine galvanische Trennung zwischen externem Anschluss des Multifunktionsgehäuse 1 und der übrigen Schaltung erfolgt. Das Volumen der Schaltung der Messeinheit erhöht sich hierdurch nicht.

Die elektrische Sicherheit wird ferner verbessert, weil der Datentransfer und das Aufladen des Akkus 15 der Messeinheit 2 nur im geschlossenen Zustand möglich sind.

Die Anzeige der Messeinheit 2 kann aufgrund des transparenten Fensters 14 des Multifunktionsgehäuses 1 zur Anzeige von Statusinformationen genutzt werden. Eine separate Anzeige für das Multifunktionsgehäuse 1 ist daher nicht unbedingt notwendig, aber möglich.

Die Messeinheit 2 braucht keine eigene Ladeschaltung zu haben, wenn diese bereits in das Multifunktionsgehäuse 1 integriert ist, sodass das Volumen der Messeinheit 2 durch die Ladeschaltung auch nicht vergrößert wird.

Grundsätzlich ist die Erfindung besonders für eine Messeinheit 2 geeignet, wenn für den Entwurf dieser Messeinheit 2 einer oder mehrere der folgenden Aspekte relevant sind:
- Die Messeinheit 2 verfügt über einen Akku und ist aufladbar;
- Die Messeinheit 2 verfügt über externe Schnittstellen zur Datenübertragung, z.B. USB;
- Die Messeinheit 2 verfügt über Messfunktionen, für die Elektroden notwendig sind;
- Das Volumen der Messeinheit 2 sollte möglichst klein sein;
- Die Messeinheit 2 muss eine hohe elektrische Sicherheit aufweisen.

Das Material des Multifunktionsgehäuses 1 kann so gewählt werden, dass die Messeinheit 2 vor Wasser, Feuer, UV-Strahlung oder Stürzen aus großer Höhe geschützt ist.

Die beschriebene Erfindung eignet sich auch zur Kalibration der Messeinheit 2, da die Erfindung automatisch eine Abschirmung gegen eine Reihe von bei der Kalibration störenden Umgebungseinflüssen darstellt, wenn die Messeinheit 2 eingelegt und der Deckel 3 geschlossen ist. Zu diesen Einflüssen gehören unter anderem:
- Schutz vor versehentlichem Berühren der Messeinheit 2 während eines Kalibrationsvorgangs;
- Schutz vor unerwünschten Luftzügen, beispielsweise durch geöffnete Fenster in der Umgebung;
- Schutz vor bestimmten Anteilen des elektromagnetischen Spektrums, wenn das Fenster 14 des Multifunktionsgehäuses 1 mit einer entsprechenden Filterfunktion ausgestattet ist;
- Dämpfung von kurzzeitigen Temperaturschwankungen, Luftdruck- oder Luftfeuchtigkeitsänderungen bei geschlossenem Multifunktionsgehäuse 1.

Je nach Art der zu kalibrierenden Sensoren der Messeinheit 2 kann die Erfindung in der bereits beschriebenen Form für die Kalibration ausreichend sein oder sie kann um die folgenden Eigenschaften für Sensoren mit bestimmten Anforderungen an den Kalibrationsvorgang erweitert werden:
- Eine Kombination des Multifunktionsgehäuses 1 mit einer vollständig lichtabsorbierenden Einlage für das transparente Fenster 14 oder Austausch des transparenten Fensters 14 gegen ein abgedunkeltes Fenster, sodass die Kalibration der Messeinheit 2 (z.B. von in bestimmten Ausführungen vorhandenen optischen Sensoren) im Dunkeln ohne Umgebungslicht stattfinden kann;
- Eine elektromagnetische Abschirmung des Multifunktionsgehäuses 1, um Sensoren, die sensitiv für elektromagnetische Störungen sind, störungsfrei kalibrieren zu können;
- Eine Temperaturstabilisierung des Multifunktionsgehäuses 1 oder Integration von Heiz- und/oder Kühlelementen, wenn eine oder mehrere definierte Temperaturen für den Kalibrationsprozess der Messeinheit 2 erzeugt werden sollen oder die Messeinheit 2 gegen äußere Temperaturschwankungen abgeschirmt sein soll;
- Dämpfung von Stößen oder Vibrationen (z.B. durch ein schweres vorbeifahrendes Fahrzeug oder einen Zug), um die Kalibration von vibrationsempfindlichen Sensoreinheiten zu ermöglichen.

Falls dies für die Art der in die Messeinheit 2 integrierten Sensoren notwendig ist, kann die Messeinheit 2 vor Einlegen in das Multifunktionsgehäuse 1 auch mit weiteren Hilfsmitteln für die Kalibration kombiniert werden. Ein Beispiel hierfür ist das Einlegen eines definierten Materials mit bestimmten optischen Eigenschaften oder optischer Filter in die Messeinheit 2, um die Kalibration der optischen Sensoren zu ermöglichen, falls die Messeinheit 2 über solche Sensoren verfügt.

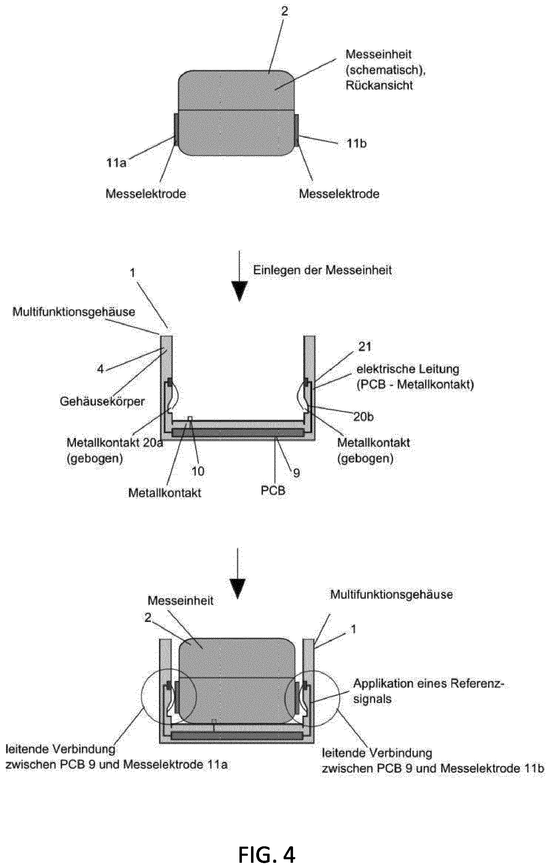

Ebenso kann das Multifunktionsgehäuse 1, wie in Figur 4 gezeigt, innen über elektrische Anschlüsse verfügen, die z.B. so in die Auflagefläche der Messeinheit 2 im Gehäuseboden oder in die Wände eingelassen sind, so dass elektrische Referenzsignale auf die Messelektroden 11a, b der Messeinheit 2 appliziert werden können. In der gezeigten Ausführung werden zur Herstellung des elektrischen Kontakts zwischen Multifunktionsgehäuse 1 und Messelektroden 11a, b gebogene Metallkontakte 20a, b verwendet, die sich beim Einlegen der Messeinheit 2 in das Multifunktionsgehäuse gegen die Messelektroden 11a, b drücken.

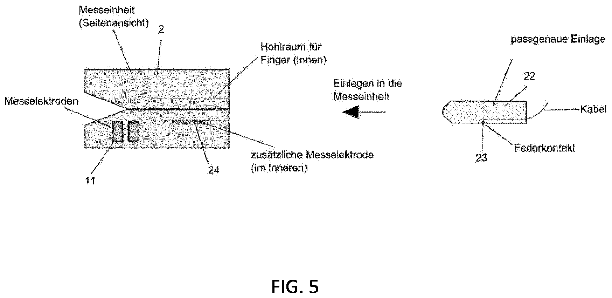

Sofern die Messeinheit aufklappbar ist und auch im Inneren über Elektroden verfügt, kann zur Applikation der Referenzsignale auch wie in Figur 5 gezeigt ein Hilfsmittel mit elektrischen Kontakten in die Messeinheit 2 eingelegt werden, das dann über elektrische Anschlüsse mit dem PCB 9 verbunden wird. Im gezeigten Ausführungsbeispiel handelt es sich um eine passgenaue Einlage 22 für den Innenraum der aufklappbaren Messeinheit 2, die über einen Federkontakt 23 verfügt, der zur Kontaktierung einer Messelektrode 24 im Innenraum der Messeinheit 2 verwendet wird und über den ein Referenzsignal appliziert werden kann.

Die elektrischen Referenzsignale können dabei von der Schaltung 8 erzeugt werden, die sich auf dem PCB 9 befindet, das in den Gehäuseboden des Multifunktionsgehäuses 1 eingelassen ist. Denkbar ist jedoch auch, dass die Signale beispielsweise über den Konnektor 7 des Multifunktionsgehäuses 1 (z.B. USB) eingespeist werden und dabei von einer externen Einheit wie einem Personal Computer 18 generiert werden.

Verfügt die Messeinheit 2 über eine Vorrichtung zur Bioimpedanzmessung, dann ist es nützlich, wenn in das Multifunktionsgehäuse 1 bzw. die integrierte Schaltung 8 auf dem PCB 9 technische Impedanzen integriert sind, die über elektrische Anschlüsse mit den Elektroden der Messeinheit 2 verbunden werden können, sodass die Vorrichtung zur Bioimpedanzmessung der Messeinheit 2 mit den definierten technischen Impedanzen der Schaltung 8 kalibriert werden kann.

Zusätzlich kann das Multifunktionsgehäuse 1 innen über weitere elektrische Anschlüsse verfügen, über die zusätzliche Hilfsmittel für die Kalibration mit Strom versorgt werden. Ein Beispiel hierfür ist ein Piezoelement, das über eine entsprechende Vorrichtung mit einem oder mehreren Temperatursensoren der Messeinheit 2 (sofern vorhanden) verbunden wird und zur Temperaturkalibration eingesetzt wird.

Im Falle eines komplexen Kalibrationsprozesses mit mehreren Schritten kann der Kalibrationsprozess durch Software unterstützt werden, die auf dem Mikrocontroller 16 der Messeinheit 2, auf einem auf dem PCB 9 des Multifunktionsgehäuses 1 befindlichem Mikrocontroller oder auf einer externen Einheit, die über den Konnektor des Multifunktionsgehäuses 1 anschlossen wird, ausgeführt wird. Es ist auch eine Kombination dieser Möglichkeiten denkbar.

Der Kalibrationsprozess selber kann entweder vollständig automatisiert sein oder halb-automatisch mit Einbeziehung des Nutzers erfolgen. Der Status der Kalibration kann dem Nutzer sowohl über optische als auch akustische Signale (z.B. ein bestimmter Signalton) mitgeteilt werden.

Unabhängig von der Kalibration kann es je nach Art der Messeinheit 2 sinnvoll oder sogar notwendig sein, die Funktionalität der Messeinheit 2 vor der Durchführung der eigentlichen Messung zu prüfen. Dies ist insbesondere bei Messeinheiten 2 wichtig, bei denen es essentiell ist, die Wahrscheinlichkeit für das Anzeigen von fehlerhaften Messergebnissen (z.B. durch defekte Sensoreinheiten) zu minimieren.

In begrenztem Umfang kann die Funktionalität der Sensorik einer Messeinheit 2 in vielen Fällen vor der Messung einer grundsätzlichen Plausibilitätskontrolle unterzogen werden, die in der Messeinheit 2 selber erfolgt. Aber eine umfassende Kontrolle der Funktionalität ist häufig ohne externe Referenzsignale nicht möglich. Beispiele für Fälle, in denen die Funktionalität nicht vollständig ohne externe Referenzsignale geprüft werden kann und bei denen die vorgenannten Mittel zur Kalibration dementsprechend sehr nützlich für die Funktionskontrolle sind, sind wie folgt:
- Bei einem Temperatursensor, der die Körpertemperatur des Nutzers messen soll, kann vor der Messung geprüft werden, ob der von der Sensoreinheit zurückgelieferte Messwert in einem plausiblen Bereich liegt; aber die Reaktion des Sensors auf unterschiedliche Temperaturen kann nicht ohne weitere Hilfsmittel geprüft werden. Ist wie vorher beschrieben ein Piezoelement in die Erfindung integriert, dann kann mit diesem die Antwort des Temperatursensors auf eine definierte Temperatur geprüft werden;
- Verfügt die Messeinheit 2 über eine Vorrichtung zur ECG-Messung, dann befinden sich die Leitungen der ECG-Schaltung zu den Elektroden vor der Messung in einem offenen Zustand. Ohne die Möglichkeit, ein externes Testsignal über die Elektroden einzuspeisen, kann die ECG-Schaltung auch nicht vollständig geprüft werden. Zur Prüfung kann in diesem Fall beispielsweise ein synthetisches, von der integrierten Schaltung 8 wie bei der Kalibration generiertes Testsignal an den Elektroden der Messeinheit 2 appliziert werden;
- Verfügt die Messeinheit 2 über Vorrichtungen zur Messung elektrischer Eigenschaften des Nutzers (z.B. für Bioimpedanzmessungen), dann können die für die Kalibration in die integrierte Schaltung 8 eingesetzten technischen Bauteile mit definierten elektrischen Eigenschaften (z.B. technische Impedanzen) verwendet werden um zu prüfen, ob die Messeinheit 2 die elektrischen Eigenschaften dieser Bauteile im Rahmen einer Toleranz korrekt misst.

Wird die Erfindung, wie zuvor beschrieben, so verändert, dass das Fenster 14 im Deckel 3 intransparent ist, dann steht außerdem eine definierte Testumgebung für mögliche optische Sensoreinheiten zur Verfügung. Werden z.B. Photodioden als optische Sensoren in der Messeinheit 2 verwendet, dann kann u.a. deren Dunkelstrom geprüft werden.

Ebenso ist denkbar, die Erfindung um integrierte Lichtquellen (z.B. LEDs) zu erweitern, die eine Prüfung der optischen Sensoren der Messeinheit 2 unabhängig davon, ob die Messeinheit 2 selber über Lichtquellen verfügt und ob diese funktionieren, ermöglichen. Genauso kann die Erfindung um optische Sensoren erweitert werden, die die Lichtquellen der Messeinheit 2, unabhängig von den optischen Sensoren der Messeinheit 2, prüfen.

Verfügt die Messeinheit 2 über aktive Bauteile, die z.B. einen Strom oder eine Spannung generieren (z.B. für Bioimpedanzmessungen), dann können diese von der Messeinheit 2 generierten Signale über Erweiterungen der Erfindung mit Vorrichtungen zur Strom- und/oder Spannungsmessung geprüft werden.

Es ist wichtig darauf hinzuweisen, dass die beschriebenen Erweiterungen des Multifunktionsgehäuses 1 zur Prüfung der Funktionalität der Messeinheit 2 zum Teil zwar ähnlich oder funktionell sogar identisch zu den vorher beschriebenen Vorrichtungen zur Kalibration sind, es sich aber bei Prüfung und Kalibration um zwei unterschiedliche Anwendungsfälle für diese Vorrichtungen handelt. Diese Anwendungsfälle können, aber müssen nicht beide für die Messeinheit 2 realisiert sein. Beispielsweise sind Messeinheiten 2 denkbar, die nicht beim Nutzer kalibriert werden müssen, aber für die trotzdem ein regelmäßiger Funktionstest beim Nutzer sinnvoll ist, um erst beim Nutzer auftretende technische Defekte erkennen zu können. Bei Erkennung eines Defekts kann der Nutzer mittels einer Warnmeldung über diesen Defekt informiert werden.

Kalibrations- und/oder Funktionstest der Messeinheit 2 mit dem Multifunktionsgehäuse 1 können dabei aufgrund der kompakten Bauweise in regelmäßigen Abständen beim Endanwender erfolgen. Es ist jedoch ebenso denkbar, dass das Multifunktionsgehäuse 1 bereits bei der Produktion der Messeinheit 2 zur Initialisierung und Kalibration der Messeinheit 2 und durch die Möglichkeiten zur Funktionskontrolle auch zur Qualitätssicherung während der Produktion verwendet wird.

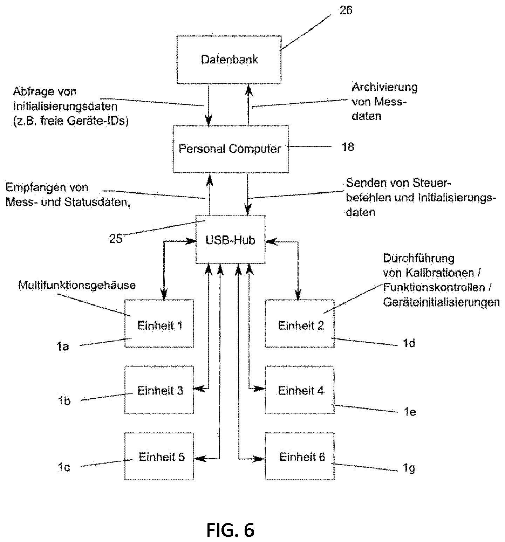

Ein Einsatz des Multifunktionsgehäuses 1 in der Produktion, bei dem mehrere erfindungsgemäße Multifunktionsgehäuse 1 parallel betrieben werden, ist in Figur 6 dargestellt. Dort werden mehrere Multifunktionsgehäuse 1a - 1f über deren USB-Schnittstelle 17 mit an einen USB-Hub 25 angeschlossen, der wiederum mit einem Personal Computer 18 verbunden ist, wobei erfindungsgemäß auch eine andere Kommunikationsschnittstelle als USB denkbar ist. Der Personal Computer 18 sendet Steuerbefehle an die Multifunktionsgehäuse 1a-1f bzw. die eingelegten Messeinheiten 2 sowie zusätzliche Initialisierungsinformationen (z.B. die für die Messeinheit 2 zu verwendende Geräte-Identifikationsnummer), falls eine Initialisierung der Messeinheiten 2 notwendig ist. Die Ergebnisse der Kalibration bzw. des Funktionstests werden von den Multifunktionsgehäusen 1a-1f an den Personal Computer 18 gesendet, der die Resultate sammeln, bewerten und aufbereiten kann. Ebenso ist es möglich, dass die Ergebnisse für die weitere Verwendung in einer Datenbank 26 gespeichert werden.

### Bezugszeichenliste:

- 1, 1a-f: Multifunktionsgehäuse
- 2: Messeinheit
- 3: Deckel
- 4: Gehäusekörper
- 5: Kontaktstift
- 6: Gegenstück zu Kontaktstift 5
- 7: Konnektor
- 8: integrierte Schaltung
- 9: PCB (Printed Circuit Board)
- 10: Metallkontakte
- 11, 11a-b: Messelektroden
- 12: Display
- 13: Bedienknöpfe
- 14: Fenster
- 15: Akku
- 16: Mikrocontroller
- 17: USB-Anschluss
- 18: Personal Computer (PC)
- 19: Akku-Lade-IC
- 20: gebogener Metallkontakt
- 21: Elektrische Leitung
- 22: passgenaue Einlage für die Messeinheit
- 23: Federkontakt der passgenauen Einlage
- 24: Messelektroden im Inneren der Messeinheit 2
- 25: USB-Hub
- 26: Datenbank

## Patentansprüche

1. Multifunktionsgehäuse (1) mit einer entnehmbar darin angeordneten medizinischen Messeinheit (2) mit integrierter Sensorik, welches einen Gehäusekörper (4) und einen beweglichen Deckel (3) aufweist, wobei der Gehäusekörper (4) mittels des Deckels (3) verschließbar ist,
wobei die Messeinheit (2) Messelektroden (11) für Bioimpedanz- oder ECG-Messungen aufweist, und
das Multifunktionsgehäuse (1) über eine integrierte Schaltung (8) verfügt und mindestens eine Schnittstelle aufweist, die eine Interaktion zwischen der Umgebung und der Messeinheit (2) ermöglicht, wobei die mindestens eine Schnittstelle als Ladeschnittstelle ausgebildet ist,
**dadurch gekennzeichnet, dass**
am Deckel (3) Mittel vorgesehen sind, um zu detektieren, ob das Multifunktionsgehäuse (1) verschlossen ist, und dass ein Laden der Messeinheit (2) nur möglich ist, wenn das Multifunktionsgehäuse (1) geschlossen ist.

2. Multifunktionsgehäuses (1) nach Anspruch 1, **dadurch gekennzeichnet, dass** mindestens eine Schnittstelle als Datenkommunikationsschnittstelle ausgebildet ist.

3. Multifunktionsgehäuses (1) nach einem der vorgenannten Ansprüche, **dadurch gekennzeichnet, dass** das Multifunktionsgehäuse (1) ein transparentes Fenster (14) aufweist.

4. Multifunktionsgehäuses (1) nach Anspruch 3, **dadurch gekennzeichnet, dass** das transparente Fenster (11) eine Lupenfunktion aufweist.

5. Multifunktionsgehäuses (1) nach Anspruch 2, 3 oder 4, **dadurch gekennzeichnet, dass** die Datenkommunikationsschnittstelle drahtlos ausgebildet ist.

6. Multifunktionsgehäuses (1) nach einem der vorgenannten Ansprüche, **dadurch gekennzeichnet, dass** das Multifunktionsgehäuse (1) über eine Tonausgabe, insbesondere eine Sprachausgabe, verfügt.

7. Multifunktionsgehäuses (1) nach einem der vorgenannten Ansprüche, **dadurch gekennzeichnet, dass** am Multifunktionsgehäuse (1) Mittel zur Klimatisierung des Innenraums des Multifunktionsgehäuses (1) vorgesehen sind.

8. Multifunktionsgehäuses (1) nach einem der vorgenannten Ansprüche, **dadurch gekennzeichnet, dass** der Deckel (3) mittels eines Klappmechanismus beweglich ist.

9. Multifunktionsgehäuses (1) nach einem der vorgenannten Ansprüche, **dadurch gekennzeichnet, dass** der Deckel (3) mittels eines Schiebemechanismus beweglich ist.

10. Multifunktionsgehäuses (1) nach einem der vorgenannten Ansprüche, **dadurch gekennzeichnet, dass** in das Multifunktionsgehäuse (1) Mittel zur Erzeugung eines optischen, thermischen und/oder elektrischen Referenzsignals vorgesehen sind.

11. Multifunktionsgehäuses (1) nach einem der vorgenannten Ansprüche, **dadurch gekennzeichnet, dass** am Multifunktionsgehäuse (1) Mittel zur optischen, thermischen und/oder elektromagnetischen Abschirmung vorgesehen sind.

12. Verfahren zur Durchführung einer Messung von physiologischen Parametern und zur Analyse der Parameter unter Verwendung eines Multifunktionsgehäuses (1) nach einem der vorgenannten Ansprüche, bei dem
- die Messeinheit (2) aus dem Multifunktionsgehäuse (1) entnommen wird,
- die Messung der physiologischen Parameter mit Hilfe der Messeinheit (2) durchgeführt wird,
- die Messeinheit (2) die Parameter zumindest temporär als Messdaten speichert,
- die Messeinheit (2) in das Multifunktionsgehäuse (1) eingelegt wird und
- die Messdaten von der Messeinheit (2) an das Multifunktionsgehäuse (1) zu Analyse übertragen werden oder vom Multifunktionsgehäuse (1) an eine übergeordnete Auswerteeinheit zur Analyse durchgeleitet werden.

## Claims

1. Multi-function housing (1) with a medical measuring device (2) removably arranged therein with integrated sensor technology, which has a housing body (4) and a movable lid (3), the housing body (4) being closable by means of the lid (3),
wherein the measuring device (2) has measuring electrodes (11) for bioimpedance or ECG measurements, and the multi-function housing (1) has an integrated circuit (8) and has at least one interface which enables interaction between the environment and the measuring device (2), wherein the at least one interface is designed as a charging interface,
**characterized in that**
means are provided on the lid (3) in order to detect whether the multi-function housing (1) is closed, and that charging of the measuring device (2) is only possible when the multi-function housing (1) is closed.

2. Multi-function housing (1) according to claim 1, **characterized in that** at least one interface is designed as a data communication interface.

3. Multi-function housing (1) according to any one of the preceding claims, **characterized in that** the multi-function housing (1) has a transparent window (14).

4. Multi-function housing (1) according to claim 3, **characterized in that** the transparent window (11) has a magnifying glass function.

5. Multi-function housing (1) according to claim 2, 3 or 4, **characterized in that** the data communication interface is wireless.

6. Multi-function housing (1) according to any one of the preceding claims, **characterized in that** the multi-function housing (1) has a sound output, in particular a voice output.

7. Multi-function housing (1) according to any one of the preceding claims, **characterized in that** means are provided on the multi-function housing (1) for air conditioning of the interior of the multi-function housing (1).

8. Multi-function housing (1) according to any one of the preceding claims, **characterized in that** the lid (3) is movable by means of a hinged mechanism.

9. Multi-function housing (1) according to any one of the preceding claims, **characterized in that** the lid (3) is movable by means of a sliding mechanism.

10. Multi-function housing (1) according to any one of the preceding claims, **characterized in that** the multi-function housing (1) incorporates means for generation of an optical, thermal and/or electrical reference signal are provided.

11. Multi-function housing (1) according to any one of the preceding claims, **characterized in that** means are provided on the multi-function housing (1) for optical, thermal and/or electromagnetic shielding are provided.

12. Method for performing a measurement of physiological parameters and for analyzing the parameters using a multi-function housing (1) according to any one of the preceding claims, wherein
- the measuring device (2) is removed from the multi-function housing (1),
- the measurement of the physiological parameters is carried out with the aid of the measuring device (2),
- the measuring device (2) stores the parameters at least temporarily as measurement data,
- the measuring device (2) is inserted into the multi-function housing (1) and
- the measurement data are transferred from the measuring device (2) to the multi-function housing (1) for analysis or are transmitted from the multi-function housing (1) to a higher-level evaluation unit for analysis.

## Revendications

1. Boîtier multifonction (1) avec un dispositif de mesure (2) médicale amovible disposée à l'intérieur avec capteurs intégrés, qui comprend un corps de boîtier (4) et un couvercle mobile (3), le corps de boîtier (4) pouvant être fermé au moyen du couvercle (3), dispositif de mesure (2) présentant des électrodes de mesure (11) pour des mesures de bioimpédance ou d'ECG, et le boîtier multifonction (1) disposant d'un circuit intégré (8) et présentant au moins une interface qui permet une interaction entre l'environnement et le dispositif de mesure (2), l'au moins une interface étant conçue comme une interface de charge,
**caractérisé en ce que**
des moyens sont prévus sur le couvercle (3) pour détecter si le boîtier multifonction (1) est fermé, et qu'un chargement du dispositif de mesure (2) n'est possible que si le boîtier multifonction (1) est fermé.

2. Boîtier multifonction (1) selon la revendication 1, **caractérisé en ce qu'**au moins une interface est conçue comme une interface de communication de données.

3. Boîtier multifonction (1) selon l'une des revendications précédentes, **caractérisé en ce que** le boîtier multifonction (1) comporte une fenêtre transparente (14).

4. Boîtier multifonction (1) selon la revendication 3, **caractérisé en ce que** la fenêtre transparente (11) présente une fonction de loupe.

5. Boîtier multifonction (1) selon la revendication 2, 3 ou 4, **caractérisé en ce que** l'interface de communication de données est conçue sans fil.

6. Boîtier multifonction (1) selon l'une des revendications précédentes, **caractérisé en ce que** le boîtier multifonction (1) dispose d'une sortie sonore, en particulier une sortie vocale.

7. Boîtier multifonction (1) selon l'une des revendications précédentes, **caractérisé en ce que** des moyens de climatisation de l'espace intérieur du boîtier multifonction (1) sont prévus sur le boîtier multifonction (1).

8. Boîtier multifonction (1) selon l'une des revendications précédentes, **caractérisé en ce que** le couvercle (3) peut être déplacé au moyen d'un mécanisme de rabattement.

9. Boîtier multifonction (1) selon l'une des revendications précédentes, **caractérisé en ce que** le couvercle (3) peut être déplacé au moyen d'un mécanisme de coulissant.

10. Boîtier multifonction (1) selon l'une des revendications précédentes, **caractérisé en ce que**, dans le boîtier multifonction (1), des moyens de génération d'un signal optique, thermique et/ou électrique signal de référence sont prévus.

11. Boîtier multifonction (1) selon l'une des revendications précédentes, **caractérisé en ce que**, sur le boîtier multifonction (1), des moyens de blindage optique, thermique et/ou électromagnétique sont prévus.

12. Méthode pour effectuer une mesure de paramètres physiologiques et pour l'analyse des paramètres en utilisant un boîtier multifonction (1) selon l'une des revendications précédentes, dans lequel
- le dispositif de mesure (2) est retirée du boîtier multifonction (1),
- la mesure des paramètres physiologiques est effectuée à l'aide du dispositif de mesure (2),
- le dispositif de mesure (2) mémorise les paramètres au moins temporairement en tant que données de mesure,
- le dispositif de mesure (2) est placée dans le boîtier multifonction (1) et
- les données de mesure sont transmises par le dispositif mesure (2) au boîtier multifonction (1) pour analyse ou sont transmises par le boîtier multifonction (1) à une unité d'évaluation supérieure pour analyse.
